# EUROPEAN PATENT SPECIFICATION

(11) **EP 2 320 865 B1**
(45) Date of publication and mention of the grant of the patent: **04.07.2012**
(21) Application number: 09780595.6
(22) Date of filing: 15.07.2009
(51) Int. Cl.: A61K 8/55, A61Q 17/04

(54) **USE OF PENTASODIUM (ETHYLENEDIAMINE)TETRAMETHYLENEPHOSPHATE FOR SUPPRESSING DISCOLORATION IN TOPICAL COMPOSITIONS**
VERWENDUNG VON PENTANATRIUM-ETHYLENDIAMINTETRAMETHYLENPHOSPHONAT ZUR VERHINDERUNG VON ENTFÄRBUNG IN KOSMETISCHEN ZUSAMMENSETZUNGEN
UTILISATION DU SEL PENTASODIQUE DE L'ACIDE ETHYLENEDIAMINE TETRAMETHYLENE PHOSPHONIQUE POUR SUPPRIMER LA DECOLORATION DANS DES COMPOSITIONS COSMETIQUES

(30) Priority: 16.07.2008 EP 08012833
(43) Date of publication of application: 18.05.2011
(73) Proprietor: DSM IP Assets B.V., 6411 TE Heerlen (NL)
(72) Inventor: MENDROK-EDINGER, Christine, 79618 Rheinfelden-Minseln (DE); SMITH, Kimberly, Mountain Lakes, NJ 07046 (US); VOLLHARDT, Jürgen, CH-4433 Ramlinsburg (CH)
(74) Representative: Berg, Katja
(86) International application number: PCT/EP2009/059021
(87) International publication number: WO 2010/007077

(56) References cited:
- EP-A1- 1 310 239
- EP-A1- 1 454 619

## Description

The present invention is concerned with the use of pentasodium (ethylenediamine)tetramethylenephosphonate for suppressing discoloration in cosmetic compositions comprising 2-phenylbenzimidazol-sulphonic acid or a salt thereof. Furthermore, the invention relates to a method for reducing discoloration of topical compositions containing 2-phenylbenzimidazol-sulphonic acid or a salt thereof comprising preparing a topical composition comprising 2-phenylbenzimidazol-sulphonic acid or a salt thereof, pentasodium (ethylenediamine)-tetramethylenephosphonate and a cosmetically acceptable carrier.

2-phenylbenzimidazol-sulphonic acid or a salt thereof as a UVB filter substrate in cosmetic light screening compositions are well known. However, in topical compositions containing 2-phenylbenzimidazol-sulphonic acid or a salt thereof discoloration has been observed on storage.

In accordance with the present invention it has surprisingly been found that the discoloration of topical compositions containing 2-phenylbenzimidazol-sulphonic acid or a salt thereof can be suppressed by the addition of pentasodium (ethylenediamine)-tetramethylenephosphonate.

Thus, in one aspect, the present invention is concerned with the use of pentasodium (ethylenediamine)tetramethylenephosphonate for suppressing discoloration in topical compositions comprising 2-phenylbenzimidazol-sulphonic acid or a salt thereof.

In another embodiment the invention is concerned with a method for reducing discoloration of topical compositions containing 2-phenylbenzimidazol-sulphonic acid or a salt thereof, comprising preparing a topical composition comprising 2-phenylbenzimidazol-sulphonic acid or a salt thereof, pentasodium (ethylenediamine)-tetramethylenephosphonate and a cosmetically acceptable carrier.

In a further aspect the invention relates to the use of a combination of pentasodium (ethylenediamine)-tetramethylenephosphonate and 2-phenylbenzimidazol-sulphonic acid or a salt thereof for the preparation of storage stable topical composition. The compositions in particular exhibit an excellent storage stability in view of preventing/suppressing discoloration.

Pentasodium (ethylenediamine)tetramethylenephosphonate (INCI Pentasodium Ethylenediamine Tetramethylene Phosphonate [CAS 7651-99-2]) is a chelating agent which is e.g. commercially available as Dequest 2046 by Monsanto.

The amount of pentasodium (ethylenediamine)tetramethylenephosphonate in all embodiments of the invention is suitably from about 0.001 to about 15 % by weight, preferably from about 0.01 to about 10 %, in particular from about 0.05 to about 5 wt.-% based on the total weight of the topical composition.

Suitable salts of 2-phenylbenzimidazol-sulphonic acid are alkali salts, such as sodium- or potassium salts, ammonium salts, morpholine salts, salts of primary, sec. and tert. amines like monoethanolamine salts, diethanolamine salts and salts of basic amino acids such as arginine, lysine, ornithine and histidine and the like. The salts can be used as such or prepared in situ by neutralizing 2-phenylbenzimidazol-sulphonic acid in the aqueous phase during the process of preparation of the topical composition.

The concentration of 2-phenylbenzimidazol sulphonic acid or a salt thereof in the topical composition according to the invention is preferably selected in the range of about 0.5 to 5 wt.-%, more preferably in the range of about 1 to 3 wt.-%.

The ratio of 2-phenylbenzimidazol sulphonic acid or a salt thereof to the pentasodium (ethylenediamine)tetramethylenephosphonate is not critical. For example the ratio is 10 to 1 to 3 to 1, such as 6 to 1.

In another aspect the present invention is concerned with storage stable topical compositions comprising pentasodium (ethylenediamine)tetramethylenephosphonate, 2-phenylbenzimidazol-sulphonic acid or a salt thereof and a cosmetically acceptable carrier. The compositions in particular exhibit an excellent storage stability in view of preventing/suppressing discoloration.

In all embodiments of the invention, the topical compositions preferably further comprise a phosphate ester surfactant. Suitable phosphate esters surfactants have the formula I

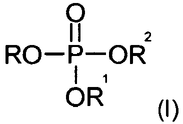

The phosphate ester surfactant has the general structure wherein R, R¹ and R² may be hydrogen, an alkyl of from 1 to about 22 carbons, preferably from about 12 to 18 carbons, or an alkoxylated alkyl of from 1 to about 22 carbons, preferably from about 12 to 18 carbons, and having 1 or more, preferably from about 2 to about 25, most preferably 2 to 12, moles ethylene oxide, with the proviso that at least one of R, R¹ and R² is an alkyl or alkoxylated alkyl as previously defined but having at least 6 alkyl carbons in said alkyl or alkoxylated alkyl group.
Monoesters in which R¹ and R² are hydrogen and R is selected from alkyls of 10 to 18 carbons and alkoxylated fatty alcohols of 10 to 18 carbons and 2 to 12 moles ethylene oxide are preferred. Among the preferred phosphate ester surfactants, mention may be made of C12-16 Pareth-6 Phosphate, C8-10 Alkyl Ethyl Phosphate, C9-15 Alkyl Phosphate, Ceteareth-2 Phosphate, Ceteareth-4 Phosphate, Ceteareth-5 Phosphate, Ceteareth-10 Phosphate, Ceteth-8 Phosphate, Ceteth-10 Phosphate, Cetyl Phosphate, C6-10 Pareth-4 Phosphate, C12-13 Pareth-10 Phosphate, C12-15 Pareth-2 Phosphate, C12-15 Pareth-3 Phosphate, C12-15 Pareth-6 Phosphate, C12-15 Pareth-8 Phosphate, C12-15 Pareth-10 Phosphate, C12-16 Pareth-6 Phosphate, DEA-Ceteareth-2 Phosphate, DEA-Cetyl Phosphate, DEA-Oleth-3 Phosphate, DEA-Oleth-5 Phosphate, DEA-Oleth-10 Phosphate, DEA-Oleth-20 Phosphate, Potassium cetyl phosphate, Deceth-9 Phosphate, Deceth-4 Phosphate and Deceth-6 Phosphate. Particular preferred phosphate ester surfactants according to the invention are cetyl phosphate, potassium cetyl phosphate and/ or DEA cetyl phosphate.

The phosphate ester surfactant is generally present in the topical compositions according to the invention in proportions ranging from 0.1 to 5 wt.-%, preferably from 0.5 to 5 wt.-%, most preferably from 2 to 3 wt.-% with respect to the total weigh of the topical composition.

The term "topical composition" as used herein refers in particular to cosmetic compositions that can be topically applied to mammalian keratinous tissue such as e.g. human skin or hair, particularly human skin.

The term "cosmetic composition" as used in the present application refers to cosmetic compositions as defined under the heading "Kosmetika" in Römpp Lexikon Chemie, 10th edition 1997, Georg Thieme Verlag Stuttgart, New York as well as to cosmetic preparations as disclosed in A. Domsch, "Cosmetic Preparations", Verlag für chemische Industrie (ed. H. Ziolkowsky), 4th edition, 1992.

The term cosmetically acceptable carrier refers to all carriers and/or excipients and/ or diluents conventionally used in topical compositions or preparations.

Preferably, the topical preparations according to the present invention are in the form of a suspension or dispersion in solvents or fatty substances, or alternatively in the form of an emulsion or micro emulsion (in particular of O/W- or W/O-type), PIT-emulsion, multiple emulsion (e. g. O/W/O- or W/O/W-type), pickering emulsion, hydrogel, alcoholic gel, lipogel, one- or multiphase solution or vesicular dispersion or other usual forms, which can also be applied by pens, as masks or as sprays. If the topical preparation is or comprises an emulsion it can also contain one or more anionic, nonionic, cationic or amphoteric surfactant(s).

In a particular embodiment, the topical compositions according to the invention are O/W emulsions. Preferably, the surfactant of the O/W emulsion are selected from the group of glycerylstearate in combination with ceteareth-20 and/ or ceteareth-25, ceteareth-6 in combination with stearylalcohol, cetylstearylalcohol in combination with PEG-40-ricinusoil and sodiumcetylstearylsulfate, triceteareth-4 phosphate, glycerylstearate, sodiumcetylstearylsulfate, lecithin trilaureth-4 phosphate, laureth-4 phosphate, stearic acid, propylenglycolstearate SE, PEG-25-hydrated ricinus oil, PEG-54-hydrated ricinus oil and/ or PEG-6 caprylic acid/caprinic acid glycerides, glyceryloleate in combination with propylenglycole, PEG-9-Stearate, PEG-20 Stearate, PEG-30-Stearate, PEG-40-stearate, PEG-100-stearate, ceteth-2, ceteth-20, polysorbate-20, polysorbate-60, polysorbate-65 and/ or polysorbate-100, glycerylstearate in combination with PEG-100 stearate, glycerylmyristate, glyceryllaurate, PEG-40-Sorbitanperoleat, Laureth-4, Ceteareth-3 and/isostearylglycerylether, cetylstearylalkohol in combination with sodium cetylstearylsulfat, laureth-23 and/ or steareth-2, glycerylstearat in combination with PEG-30 stearate, PEG-40-stearate, glycol distearate, PEG-22-dodecyl glycol Copolymer, polyglyceryl-2-PEG-4-stearat, ceteareth-12, ceteareth-20, ceteareth-30, methyl-glucosesesquistearate, steareth-10 and/ or PEG-20-stearat, steareth-2 in combination with PEG-8 distearate, steareth-21, steareth-20, isosteareth-20, PEG-45/ dodecylglycol-copolymer, methoxy-PEG-22/dodecylglycol-copolymer, PEG-40-sorbitanperoleat, PEG-40-sorbitanperisostearats, PEG-20-glycerylstearats, PEG-20-glycerylstearats, PEG-8-bee wax, polyglyceryl-2-laurate, isostearyldiglycerylsuccinat, stearamidopropyl-PG-dimoniumchloridphosphat, ceteth-20, glycerylstearate SE, triethylcitrate, PEG-20-methylglucosesesquistearat, cetylphosphate, glycerylstearatcitrate, cetearyl sulfat, sorbitansesquioleate, triceteareth-4-phosphats, trilaureth-4-phosphate, polyglycerylmethylglucosedistearate, potassium cetylphosphate, polyglyceryl-3 methylglucose distearate, isosteareth-10, polyglyceryl-2-sesquiisostearate, ceteth-10, oleth-20 and/ or isoceteth-20, glycerylstearate in combination with ceteareth-20, ceteareth-12, cetylstearylalcohol and/ or cetylpalmitate, cetylstearylalcohol in combination with PEG-20 stearate, PEG-30-stearate, PEG-40-stearate and/ or PEG-100-stearate. Particularly preferred O/W surfactants are phosphate ester surfactants such as cetyl phosphate, potassium cetyl phosphate and/ or DEA cetyl phosphate, glycerylstearate, PEG-40 stearate, PEG-100 stearate in combination with glyceryl stearate, triglycerinmethylglucosedistearate, polyglyceryl-3 methylglucose distearate, cetearylglucoside, polyethylenglycol(21)stearylether, polyethylenglycol(2)stearylether, glycerylstearatcitrate, sodium cetearyl-sulfat, setearylalkohol, stearic acid and/ or sorbitanstearate.
Particularly preferred O/W surfactants according to the invention are phosphate ester surfactant, in particular selected from cetyl phosphate, potassium cetyl phosphate and/ or DEA cetyl phosphate and/ or PEG-100 stearate in combination with glyceryl stearate. The most preferred O/W surfactants according to the invention are the phosphate ester surfactants cetyl phosphate, potassium cetyl phosphate and/ or DEA cetyl phosphate.

The oil phase of the O/W emulsions according to the invention preferably comprises oils selected from butylenglykoldicaprylat/-dicaprat, dicaprylylether, C₁₂₋₁₅-Alkylbenzoat, C₁₈₋₃₈-fatty acid triglyceride, dibutyladipate, cyclomethicone, 2-phenylethylbenzoat, isopropyl lauroyl sarkosinate as well as mixtures thereof. The amount of the oil (one or several) in the O/W emulsion according to the invention is generally selected in the range of about 0,1 bis 40 wt.-%, in particular in the range of about 1,0 to 30 wt.-%, most in particular in the range of about 5% to 20 wt.-% with respect to the total weight of the topical composition.

Preferred topical compositions according to the invention are sun care preparations.

Topical compositions in accordance with the invention can be in the form of a liquid, lotion, a thickened lotion, a gel, a cream, a milk, an ointment, a paste, a powder, a make-up, or a solid tube stick and can be optionally be packaged as an aerosol and can be provided in the form of a mousse such as a aerosol mousse, a foam or a spray foam, a spray, a stick.

The topical cosmetic compositions of the invention can also contain usual cosmetic adjuvants and additives such as e.g. further UV filter substances, preservatives, antioxidants, fatty substances, oils, water, alcohols, polyols, organic solvents, electrolytes, silicones, thickeners, film forming agents, softeners, emulsifiers, complexing agents, antifoaming agents, moisturizers, aesthetic components such as fragrances, surfactants, fillers, sequestering agents, anionic, cationic, nonionic or amphoteric polymers or mixtures thereof, propellants, acidifying or basifying agents, dyes, colorings/colorants, abrasives, absorbents, essential oils, skin sensates, astringents, antifoaming agents, pigments or nanopigments, cosmetically active ingredients or any other ingredients, carriers and/or excipients or diluents conventionally formulated into cosmetic compositions. Such cosmetic ingredients commonly used in the skin care industry, which are suitable for use in the compositions of the present invention are e.g. described in the CTFA Cosmetic Ingredient Handbook, Second Edition (1992) without being limited thereto.

The necessary amounts of the cosmetic and dermatological adjuvants and additives can - based on the desired product - easily be chosen by a skilled person in this field and will be illustrated in the examples, without being limited hereto.

Preferably, the topical compositions according to the invention comprise further UV filter substances which are preferably selected from conventional UVA and/or UVB and/ or broad spectrum UV-filter substances known to be added into topical compositions such as cosmetic or dermatological sun care products. Such UV-filter substances comprise all groups which absorb light in the range of wavelengths 400 nm to 320 nm (UVA) and 320 nm to 280 nm (UVB) or of even shorter wavelengths (UVC) and which are or can be used as cosmetically acceptable UV-filter substances. Such UV-filter substances are e.g. listed in the CTFA Cosmetic ingredient Handbook or "The Encyclopedia of Ultraviolet Filters" (ISBN: 978-1-932633-25-2) by Nadim A. Shaath.
Suitable UV-filter substances may be organic or inorganic compounds. Exemplary organic UV-filter substances encompass e.g. acrylates such as e.g. 2-ethylhexyl 2-cyano-3,3-diphenylacrylate (octocrylene, PARSOL^{®} 340), ethyl 2-cyano-3,3-diphenylacrylate; Camphor derivatives such as e.g. 4-methyl benzylidene camphor (PARSOL^{®} 5000), 3-benzylidene camphor, camphor benzalkonium methosulfate, polyacrylamidomethyl benzylidene camphor, sulfo benzylidene camphor, sulphomethyl benzylidene camphor, terephthalylidene dicamphor sulfonic acid (Mexoryl^{®} SX); Cinnamate derivatives such as e.g. ethylhexyl methoxycinnamate (PARSOL^{®} MCX), ethoxyethyl methoxycinnamate, isoamyl methoxycinnamate as well as cinnamic acid derivatives bond to siloxanes; p-Aminobenzoic acid derivatives such as e.g. p-aminobenzoic acid, 2-ethylhexyl p-dimethylaminobenzoate, N-oxypropylenated ethyl p-aminobenzoate, glyceryl p-aminobenzoate; Benzophenones such as e.g. benzophenone-3, benzophenone-4, 2,2',4,4'-tetrahydroxy-benzophenone, 2,2'-dihydroxy-4,4'-dimethoxybenzophenone; Esters of benzalmalonic acid such as e.g. di-(2-ethylhexyl) 4-methoxybenzalmalonate; Organosiloxane compounds carrying chromophore groups such as e.g. polysilicones-15 (PARSOL^{®} SLX), drometrizole trisiloxane (Mexoryl^{®} XL); Salicylate derivatives such as e.g. isopropylbenzyl salicylate, benzyl salicylate, butyl salicylate, ethylhexyl salicylate (PARSOL^{®} EHS, Neo Heliopan^{®} OS), isooctyl salicylate or homomenthyl salicylate (homosalate, PARSOL^{®} HMS, Neo Heliopan^{®} HMS); Triazine derivatives such as e.g. ethylhexyl triazone (Uvinul^{®} T-150), diethylhexyl butamido triazone (Uvasorb^{®} HEB), bis-ethylhexyloxyphenol methoxyphenyl triazine (Tinosorb^{®} S); Benzotriazole derivatives such as e.g. 2,2'-methylene-bis-(6-(2H-benzotriazole-2-yl)-4-(1,1,3,3,-tetramethylbutylhphenol (Tinosorb^{®} M); Encapsulated UV-filters such as e.g. encapsulated ethylhexyl methoxycinnamate (Eusolex^{®} UV-pearls) or microcapsules loaded with UV-filters as e.g. dislosed in EP 1471995; Dibenzoylmethane derivatives such as e.g. 4-tert.-butyl-4'-methoxydibenzoyl-methane (PARSOL^{®} 1789), dimethoxydibenzoylmethane, isopropyldibenzoylmethane; Phenylene-1,4-bis-benzimidazolsulfonic acids or salts such as e.g. 2,2-(1,4-phenylene)bis-(1H-benzimidazol-4,6-disulfonic acid) (Neoheliopan AP); Amino substituted hydroxybenzophenones such as e.g. 2-(4-diethylamino-2-hydroxybenzoyl)-benzoic acid hexylester (Aminobenzophenon, Uvinul^{®} A Plus); Benzoxazol-derivatives such as e.g. 2,4-bis-[5-1 (dimethylpropyl)benzoxazol-2-yl-(4-phenyl)-imino]-6-(2-ethylhexyl)-imino-1,3,5-triazine [Uvasorb^{®} K2A);
Inorganic UV-filter substances encompass pigments such as e.g. microparticulated Zink oxide or Titanium dioxide (e.g. commercially available as PARSOL^{®} TX) The term "microparticulated" refers to a particle size from about 5 nm to about 200 nm, particularly from about 15 nm to about 100 nm. The particles may also be coated by other metal oxides such as e.g. aluminum or zirconium oxides or by organic coatings such as e.g. polyols, methicone, aluminum stearate, alkyl silane. Such coatings are well known in the art.

In order to enhance the photostability of sun care products it may be desirable to add a photostabilizer. Exemplary photostabilizers known to a skilled person in the art encompass e.g. 3,3-diphenylacrylate derivatives such as e.g. octocrylene (PARSOL^{®} 340) or Polyester-8 (Polycrylene^{®}); Benzylidene camphor derivatives such as e.g. 4-methyl benzylidene camphor (PARSOL^{®} 5000); Benzalmalonate derivatives such as e.g. polysilicones-15 (PARSOL^{®} SLX) or diethylhexyl syringylidene malonate (Oxynex ST liquid); Dialkyl naphthalates such as diethylhexyl naphthalate (Corapan TQ) without being limited thereto. An overview on further stabilizers is e.g. given in 'SPF Boosters & Photostability of Ultraviolet Filters', HAPPI, October 2007,p. 77-83 which is included herein by reference. The photostabilizers are generally used in an amount of 0.05 to 10 wt.-% with respect to the total weigh of the topical composition.

Generally, the amount of each UV-filter substance in the topical compositions according to the invention is selected in the range of about 0.1 to 10 wt.-%, preferably in the range of about 0.2 to 7 wt.-%, most preferably in the range of about 0.5 to 5 wt.-% with respect to the total weigh of the topical composition.

The total amount of UVA-filter substance(s), in particular of butyl methoxydibenzoylmethane, in the topical compositions according to the invention is preferable selected in the range of about 2 to 8 wt.-%, in particular in the range of about 4 to 6 wt.-%, most particular in the range of about 4 to 5 wt.-% with respect to the total weight of the topical composition.

The total amount of UV-filter substances in the topical compositions according to the invention is preferably in the range of about 1 to 40 wt.-%, preferably in the range of about 5 to 30 wt.-%, in particular in the range of 20 to 30 wt.-% with respect to the total weight of the topical composition.

Preferred further UVB-filter substances to be used in the topical compositions according to the invention encompass polysilicones-15, octocrylene, ethylhexyl methoxycinnamate, ethyl hexylsalicylate and/ or homosalate.

Preferred broadband UV-filter substances to be used in the topical compositions according to the invention encompass unsymmetrical s-triazine derivatives such 2,4-Bis-{[4-(2-ethyl-hexyloxy)-2-hydroxy]-phenyl}-6-(4-methoxyphenyl-1,3,5-triazin, certain benzophenones such as e.g. 2-Hydroxy-4-methoxy-benzophenon, 2,2'-Methylen-bis-(6-(2H-benzotriazol-2-yl)-4-(1,1,3,3-tetramethyl-butyl)-phenol), and/ or titanium dioxide.

The preferred UVA-filter substance to be used in the topical compositions according to the invention is butyl methoxydibenzoylmethane.

Preferred topical compositions according to the invention further comprise one or several antioxidant(s). Suitable antioxidants for the incorporation into the topical compositions according to the invention are all antioxidant suitable for cosmetic applications. Particular preferred are water soluble antioxidants such as vitamins such as e.g. ascorbic acid as well as derivatives thereof such as e.g. ascorbyl phosphate such as Stay C (sodium ascorbyl monophosphate) from DSM Nutritional Products Ltd.

Further preferred antioxidants are BHT, vitamin E and derivatives thereof as well as vitamin A and derivatives thereof.

The amount of antioxidant (one or several) in the topical compositions according to the invention is preferably selected in the range of about 0.001 to 30 wt.-%, in particular in the range of about 0.05 to 20 wt.-%, most particular in the range of about 0.1 to 10 wt.-%, with respect to the total amount of the topical composition.

If a vitamin E derivative is used in the topical compositions according to the invention preferably tocopheryl acetate is used. Tocopheryl acetate may be present in the topical preparations in an amount from about 0.05 to 25 wt.-%, in particular 0.5 to 5 wt.-%. Another vitamin E derivative of interest is tocopheryl linoleate. Tocopheryl linoleate may be present in the skin care composition in an amount from about 0.05 to 25 wt.-% in particular 0.5 to 5 wt.-%.

Vitamin A and/or its derivatives in particular retinoid derivatives such as retinyl palmitate or retinyl propionate is preferably used in the topical preparations according to the invention in an amount of 0.01 to 5 wt.-%, in particular 0.01 to 0.3 wt.-%.

Preferably, the topical compositions according to the invention further comprise a fatty alcohol, such as in particular cetyl alcohol, cetearyl alcohol and/ or behenyl alcohol. The total amount of one or several fatty alcohols on the topical compositions according to the invention is preferably selected in the range of about 0.1 to 10.0 wt.-%, in particular in the range of about 0.5 to 6.0 wt.-% with respect to the total weight of the topical composition.

Suitable cosmetically active ingredients according to the invention encompass agents suitable for skin lightening; tanning prevention; self tanning; treatment of hyperpigmentation; preventing or reducing acne; treatment of wrinkles, lines, atrophy and/or inflammation; treatment of cellulites (e.g. phytanic acid), firming, energizing, skin soothing, as well as agents to improve skin elasticity and skin barrier.
The cosmetically active ingredients useful herein can in some instances provide more than one benefit or operate via more than one mode of action.

Preferred examples of cosmetically active ingredients for the incorporation into topical compositions according to the invention encompass vitamin B₆, vitamin B₁₂, biotin, co-enzyme Q10, EGCG, alpha-liponic acid, phytoen, hydroxytyrosol and/or olive extract, shea butter, algae extract, cocoa butter, aloe extract, jojoba oil, echinacea extract, chamomile extract, alpha-glucosylrutin, carnitin, carnosine, natural and/ or synthetic isoflavanoids, creatin, taurin, alanine, glycyrrhetinic acid, glycyryca glabra and/ or glycyrrhiza inflata.

The cosmetically active ingredient is typically included in an amount of at least 0.001 wt. % based on the total weight of the topical preparation. Generally, an amount of about 0.001 wt. % to about 30 wt. %, preferably from about 0.001 wt. % to about 10 wt. % of an additional cosmetically active agent is used.Further preferred the topical compositions according to the invention further comprise a moisturizer. Moisturizers are chemical agents specially designed to make the external layers of the skin (epidermis) softer and more pliable by increasing its hydration (water content) which can be determined e.g. by measuring the transepidermal water loss (TEWL). Suitable moisturizer according to the invention are for example glycerin, lactic acid and/ or lactate such as in particular sodium lactate, butyleneglycol, propyleneglycol, biosaccaride gum-1, glycine soja, ethylhexyloxyglycerine, pyrrolidoncarbonic acid and/or urea.

Suitable preservatives according to the invention encompass for example formaldehyde releasing chemicals such as e.g. DMDM Hydantoin (e.g. as Glydant by Lonza), iodopropylbutylcarbamate (e.g. as Glycacil-L, Glycacil-S by Lonza and/ or Dekaben LMB by Jan Dekker), parabens (e.g. p-hydroxybenzoic acid alkyl ester such as methyl-, ethyl-, propyl- and/ or butylparaben), phenoxyethanol, ethanol, benzoic acid without being limited thereto.

Preferably, the topical compositions according to the invention comprise a thickener in particular if the topical composition is in the form of an emulsion to assist in making the consistency of a product suitable. Preferred thickeners are aluminiumsilicates, xanthan gum, hydroxypropylmethylcellulose, polyacrylates such as carbopole^{®} (e.g. Carbopole 980, 981, 1382, 2984, 5984) or mixtures thereof. Further preferred thickeners encompass acrylate/C₁₀₋₃₀ alkyl acrylate copolymers (such as e.g. Pemulen TR 1, Pemulen TR 2, Carbopol 1328 by. NOVEON) as well as Aristoflex AVC (INCI: Ammonium Acryloyldimethyltaurate/VP Copolymer).

Of course, one skilled in this art will take care to select the above mentioned optional additional compound or compounds and/or their amounts such that the advantageous properties intrinsically associated with the combination in accordance with the invention are not, or not substantially, detrimentally affected by the envisaged addition or additions. The cosmetic and/ or dermatological compositions according to the invention have a pH in the range of 3-10, preferably in the range of pH of 4-8, most preferred in the range of pH 4-7.

The following example is provided to further illustrate the compositions and effects of the present invention. These examples are illustrative only and are not intended to limit the scope of the invention in any way.

### Example

A formulation comprising pentasodium (ethylenediamine)tetramethylenephosphonate has been prepared as outlined above and stored for 4 months at 43°C. As reference the same composition comprising Disodium Ethylenediamine Tetra Acetic Acid (EDTA) as chelating agent has been prepared.

| **Phase** | **INCI Name** | **Wt.-%** |
|---|---|---|
| **A** | Butyl Methoxydibenzoylmethane (Avobenzone; USAN) | 4.00 |
| | Polysilicone-15 | 3.00 |
| | Glyceryl Myristate | 4.00 |
| | Cetyl Alcohol | 2.00 |
| | BHT | 0.05 |
| | Dimethicone | 2.00 |
| | Phenoxyethanol & Methylparaben & Ethylparaben & Butylparaben & Propylparaben & Isobutylparaben | 0.80 |
| | Potassium Cetyl Phosphate | 3.00 |
| | Mineral Oil | 3.60 |
| | Diisopropyl Sebacate | 12.00 |
| | Dibutyl Adipate | 10.00 |
| | VP/Eicosene Copolymer | 2.00 |
| **B** | Glycerin | 3.00 |
| | Xanthan Gum | 0.30 |
| | Aqua | Ad 100 |
| | Pentasodium Ethylenediamine Tetramethylene Phosphonate | 0.50 |
| **C** | Triethanolamine | 0.30 |
| **D** | Aqua | 10.00 |
| | Triethanolamine | 3.30 |
| | Phenylbenzimidazole Sulfonic Acid (Ensulizole; USAN) | 4.00 |

### Procedure

1 Heat part A to 85°C while stirring.
2 Heat part B to 80°C and add to part A while stirring and homogenizing the emulsion.
3 Cool down the emulsion to 55°C and add part C.
   Be sure that the pH of the emulsion is 7.0. If traces remain, add small quantities of the used neutralizing base until the pH-value is at least 7.0.
4 Be sure that the pH of PARSOL® HS solution is 7.0. If traces remain, add small quantities of the used neutralizing base until the particles are dissolved.
   Now add part D and homogenize intensively again.
   Then cool down to ambient temperature while mixing the emulsion.
   It is generally recommended to use vacuum while producing the emulsion.

Surprisingly, the exchange of the chelating agent EDTA (Disodium Ethylenediamine Tetra Acetic Acid) by Dequest 2046 (Pentasodium Ethylenediamine tetramethylene Phosphate) solved the problem of discoloration. The color of the formulation stayed cream-white over the whole storage time while the formulation comprising EDTA turned yellow.

## Claims

1. Use of pentasodium (ethylenediamine)tetramethylenephosphonate for suppressing discoloration in topical compositions comprising 2-phenylbenzimidazol-sulphonic acid or a salt thereof.

2. The use according to claim 1, wherein the topical composition further comprises a phosphate ester surfactant.

3. The use according to claim 2, wherein the phosphate ester surfactant is selected from cetyl phosphate, potassium cetyl phosphate and/ or DEA cetyl phosphate.

4. The use according to any one of claims 1 to 3, wherein the amount of the pentasodium (ethylenediamine)tetramethylenephosphonate is in the range of about 0.05 to 5 wt.-% based on the total weight of the by topical composition.

5. The use according to any one of claims 1 to 4, wherein the amount of the hydrophilic benzimidazole type UVB filter substance is selected in the range of about 1 to 3 wt.-%.

6. The use according to any one of claims 1 to 5, wherein the topical composition comprises at least one further UVA and/or UVB and/ or broad spectrum UV-filter substance.

7. The use according to claim 6, wherein the at least one further UVA and/or UVB and/ or broad spectrum UV-filter substance is selected from polysilicones-15, octocrylene, ethylhexyl methoxycinnamate, ethyl hexylsalicylate, homosalate, 2,4-Bis-{[4-(2-ethyl-hexyloxy)-2-hydroxy]-phenyl}-6-(4-methoxyphenyl)-1,3,5-triazine, 2-Hydroxy-4-methoxy-benzophenon, 2,2'-Methylen-bis-(6-(2H-benzotriazol-2-yl)-4-(1,1,3,3-tetramethyl-butyl)-phenol), titanium dioxide and/ or butyl methoxydibenzoylmethane.

8. Method for reducing discoloration of topical compositions containing 2-phenylbenzimidazol-sulphonic acid or a salt thereof, comprising preparing a topical composition comprising 2-phenylbenzimidazol-sulphonic acid or a salt thereof, pentasodium (ethylenediamine)-tetramethylenephosphonate and a cosmetically acceptable carrier.

9. The method according to claim 8, wherein the topical composition further comprises a phosphate ester surfactant.

10. The method according to claim 9, wherein the phosphate ester surfactant is selected from cetyl phosphate, potassium cetyl phosphate and/ or DEA cetyl phosphate.

11. The method according to any one of claims 8 to 10, wherein the amount of the pentasodium (ethylenediamine)tetramethylenephosphonate is in the range of about 0.05 to 5 wt.-% based on the total weight of the by topical composition.

12. The method according to any one of claims 8 to 11, wherein the amount of the 2-phenylbenzimidazol-sulphonic acid or a salt thereof is selected in the range of about 1 to 3 wt-%.

13. The method according to any one of claims 8 to 12, wherein the topical composition comprises at least one further WA and/or UVB and/ or broad spectrum UV-filter substance.

## Patentansprüche

1. Verwendung von Pentanatrium (Ethylendiamin)tetramethylenphosphonat zum Unterdrücken von Verfärbung in topischen Zusammensetzungen, die 2-Phenylbenzimidazolsulfonsäure oder ein Salz davon umfassen.

2. Verwendung nach Anspruch 1, wobei die topische Zusammensetzung weiterhin ein Phosphatestertensid umfasst.

3. Verwendung nach Anspruch 2, wobei das Phosphatestertensid aus der Reihe Cetylphosphat, Kaliumcetylphosphat und/oder DEA-Cetylphosphat ausgewählt ist.

4. Verwendung nach einem der Ansprüche 1 bis 3, wobei die Menge an Pentanatrium (Ethylendiamin)tetramethylenphosphonat im Bereich von ungefähr 0,05 bis 5 Gew.-% in Bezug auf das Gesamtgewicht der topischen Zusammensetzung beträgt.

5. Verwendung nach einem der Ansprüche 1 bis 4, wobei die Menge an UVB-Filtersubstanz des hydrophilen Benzimidazoltyps im Bereich von ungefähr 1 bis 3 Gew.-% ausgewählt ist.

6. Verwendung nach einem der Ansprüche 1 bis 5, wobei die topische Zusammensetzung mindestens eine weitere UVA- und/oder UVB- und/oder Breitspektrum-UV-Filtersubstanz umfasst.

7. Verwendung nach Anspruch 6, wobei die mindestens eine weitere UVA- und/oder UVB- und/oder Breitspektrum-UV-Filtersubstanz aus der Reihe Polysilikone-15, Octocrylen, Ethylhexylmethoxycinnamat, Ethylhexylsalicylat, Homosalat, 2,4-Bis{[4-(2-ethylhexyloxy)-2-hydroxy]phenyl}-6-(4-methoxyphenyl)-1,3,5-triazin, 2-Hydroxy-4-methoxybenzophenon, 2,2'-Methylenbis(6-(2H-benzotriazol-2-yl)-4-(1,1,3,3-tetramethylbutyl)phenol), Titandioxid und/oder Butylmethoxydibenzoylmethan ausgewählt ist.

8. Verfahren zur Verringerung der Verfärbung in topischen Zusammensetzungen, die 2-Phenylbenzimidazolsulfonsäure oder ein Salz davon enthalten, bei dem eine topische Zusammensetzung, die 2-Phenylbenzimidazolsulfonsäure oder ein Salz davon, Pentanatrium(Ethylendiamin)tetramethylen-phosphonat und einen kosmetisch unbedenklichen Träger umfasst, hergestellt wird.

9. Verfahren nach Anspruch 8, wobei die topische Zusammensetzung weiterhin ein Phosphatestertensid umfasst.

10. Verfahren nach Anspruch 9, wobei das Phosphatestertensid aus der Reihe Cetylphosphat, Kaliumcetylphosphat und/oder DEA-Cetylphosphat ausgewählt ist.

11. Verfahren nach einem der Ansprüche 8 bis 10, wobei die Menge an Pentanatrium (Ethylendiamin)tetra-methylenphosphonat im Bereich von ungefähr 0,05 bis 5 Gew.-% in Bezug auf das Gesamtgewicht der topischen Zusammensetzung beträgt.

12. Verfahren nach einem der Ansprüche 8 bis 11, wobei die Menge an 2-Phenylbenzimidazolsulfonsäure oder einem Salz davon im Bereich von ungefähr 1 bis 3 Gew.-% ausgewählt ist.

13. Verfahren nach einem der Ansprüche 8 bis 12, wobei die topische Zusammensetzung mindestens eine weitere UVA- und/oder UVB- und/oder Breitspektrum-UV-Filtersubstanz umfasst.

## Revendications

1. Utilisation d'(éthylènediamine)tétraméthylène-phosphonate de pentasodium pour supprimer la décoloration dans des compositions topiques comprenant l'acide 2-phénylbenzimidazol-sulfonique ou un sel de celui-ci.

2. Utilisation selon la revendication 1, **caractérisée en ce que** la composition topique comprend en outre un tensioactif d'ester de phosphate.

3. Utilisation selon la revendication 2, **caractérisée en ce que** le tensioactif d'ester de phosphate est choisi parmi le cétylphosphate, le cétylphosphate de potassium et/ou le cétylphosphate de DEA.

4. Utilisation selon l'une quelconque des revendications 1 à 3, **caractérisée en ce que** la quantité d' (éthylènediamine) tétraméthylène-phosphonate de pentasodium est dans la gamme d'environ 0,05 à 5 % en poids sur la base du poids total de la composition topique.

5. Utilisation selon l'une quelconque des revendications 1 à 4, **caractérisée en ce que** la quantité du filtre UVB de type benzimidazole hydrophile est choisie dans la gamme d'environ 1 à 3 % en poids.

6. Utilisation selon l'une quelconque des revendications 1 à 5, **caractérisée en ce que** la composition topique comprend au moins un autre filtre UVA et/ou UVB et/ou UV à large spectre.

7. Utilisation selon la revendication 6, **caractérisée en ce que** ledit au moins un autre filtre UVA et/ou UVB et/ou UV à large spectre est choisi parmi les polysilicones-15, l'octocrylène, le méthoxycinnamate d'éthylhexyle, l'hexylsalicylate d'éthyle, l'homosalate, la 2,4-bis-{[4-(2-éthylhexyloxy)-2-hydroxy]-phényl}-6-(4-méthoxyphényl)-1,3,5-triazine, la 2-hydroxy-4-méthoxy-benzophénone, le 2,2'-méthylène-bis-(6-(2H-benzotriazol-2-yl)-4-(1,1,3,3-tétraméthyl-butyl)-phénol), le dioxyde de titane et/ou le méthoxydibenzoylméthane de butyle.

8. Méthode de réduction de la décoloration des compositions topiques contenant l'acide 2-phénylbenzimidazol-sulfonique ou un sel de celui-ci, comprenant la préparation d'une composition topique comprenant l'acide 2-phénylbenzimidazol-sulfonique ou un sel de celui-ci, l'(éthylènediamine)-tétraméthylènephosphonate de pentasodium et un support cosmétiquement acceptable.

9. Méthode selon la revendication 8, **caractérisée en ce que** la composition topique comprend en outre un tensioactif d'ester de phosphate.

10. Méthode selon la revendication 9, **caractérisée en ce que** le tensioactif d'ester de phosphate est choisi parmi le cétylphosphate, le cétylphosphate de potassium et/ou le cétylphosphate de DEA.

11. Méthode selon l'une quelconque des revendications 8 à 10, **caractérisée en ce que** la quantité d'(éthylènediamine)tétraméthylènephosphonate de pentasodium est dans la gamme d'environ 0,05 à 5 % en poids sur la base du poids total de la composition topique.

12. Méthode selon l'une quelconque des revendications 8 à 11, **caractérisée en ce que** la quantité d'acide 2-phénylbenzimidazol-sulfonique ou d'un sel de celui-ci est choisie dans la gamme d'environ 1 à 3 % en poids.

13. Méthode selon l'une quelconque des revendications 8 à 12, **caractérisée en ce que** la composition topique comprend au moins un autre filtre UVA et/ou UVB et/ou UV à large spectre.
